# EUROPEAN PATENT SPECIFICATION

(11) **EP 1 575 578 B1**
(45) Date of publication and mention of the grant of the patent: **09.12.2009**
(21) Application number: 03759773.9
(22) Date of filing: 08.10.2003
(51) Int. Cl.: A61K 31/575, A61K 31/19, A61P 25/00

(54) **METHODS OF TREATING INJURIES OF THE NERVOUS SYSTEM ASSOCIATED WITH HEMORRHAGE**
VERFAHREN ZUR BEHANDLUNG VON VERLETZUNGEN DES NERVENSYSTEMS IN VERBINDUNG MIT EINER BLUTUNG
METHODES PERMETTANT DE TRAITER DE BLESSURES DU SYSTEME NERVEUX ASSOCIEES A UNE HEMORRAGIE

(30) Priority: 07.11.2002 US 425210 P; 03.03.2003 US 451615 P
(43) Date of publication of application: 21.09.2005
(73) Proprietor: REGENTS OF THE UNIVERSITY OF MINNESOTA, Minneapolis, MN 55455 (US)
(72) Inventor: STEER, Clifford, J., St. Paul, MN 55116 (US); LOW, Walter, C., Shorewood, MN 55331 (US); RODRIGUEZ, Cecilia, M., P., P-1600 Lisbon (PT); NAN, Zhenhong, Roseville, Minnesota 55113 (US)
(74) Representative: Vossius & Partner
(86) International application number: PCT/US2003/031989
(87) International publication number: WO 2004/043342

(56) References cited:
- US-B1- 6 555 141
- RODRIGUES C M P ET AL: "NEUROPROTECTION BY A BILE ACID IN AN ACUTE STROKE MODEL IN THE RAT" JOURNAL OF CEREBRAL BLOOD FLOW AND METABOLISM, RAVEN PRESS, LTD., NEW YORK, NY, US, vol. 22, no. 4, April 2002 (2002-04), pages 463-471, XP009064738 ISSN: 0271-678X
- MATSUSHITA KOHJI ET AL: "Evidence for apoptosis after intracerebral hemorrhage in rat striatum" JOURNAL OF CEREBRAL BLOOD FLOW AND METABOLISM, vol. 20, no. 2, February 2000 (2000-02), pages 396-404, XP009088843 ISSN: 0271-678X
- MATSUSHITA K ET AL: "Efficacy of caspase inhibition for intracerebral hemorrhage in rats" SOCIETY FOR NEUROSCIENCE ABSTRACTS, vol. 24, no. 1-2, 1998, page 252, XP009088844 & 28TH ANNUAL MEETING OF THE SOCIETY FOR NEUROSCIENCE, PART 1; LOS ANGELES, CALIFORNIA, USA; NOVEMBER 7-12, 1998 ISSN: 0190-5295

## Description

### BACKGROUND

Hemorrhagic stroke remains a devastating cerebrovascular event with an estimated incidence of 15 to 35 per 100,000 and a mortality rate approaching 50%. Currently, there are approximately 2.9 million survivors of stroke, most of whom are severely disabled. Data indicate that 10-15% of strokes are a result of intracerebral hemorrhage, thus accounting for about 300,000 to 450,000 stroke survivors. Major risk factors for intracerebral hemorrhage (ICH) include hypertension, trauma, arteriovenous malformations, and tumors.

Acute diseases of the central nervous system such as hemorrhagic and ischemic stroke usually result in delayed neuronal cell death. Although the precise mechanism of brain injury is not fully understood, a number of studies suggest that apoptosis, programmed cell death, may play a key role.

Injury secondary to the mass effect of the hematoma is thought to arise from tissue reaction to invasion of blood products, resulting in ischemia, edema, intense inflammation, and ultimately cell death. The mechanisms of cell death from hemorrhagic stroke are complex and appear to involve an interplay of events, ranging from necrosis to apoptosis. However, it has been recognized that after cerebral ischemia followed by reperfusion, cells exhibit several features of apoptosis including chromatin condensation, TUNEL labeling, and activation of caspases. Characteristic signs of apoptosis have also been described in the brain after hemorrhagic stroke. Nevertheless, the mechanism of neuronal loss after hemorrhagic stroke is not well documented in part because of the lack of adequate experimental models.

Ursodeoxycholic acid (UDCA) is an endogenous bile acid that has been in clinical use over the last few decades for the treatment of a variety of liver diseases. The observation that UDCA and its conjugated derivatives, such as tauroursodeoxycholic acid (TUDCA), play a unique role in modulating the apoptotic threshold in both hepatic and nonhepatic cells extended the biological role of these molecules. UDCA. and TUDCA stabilize the mitochondrial membrane to prevent several apoptotic events, including mitochondrial membrane depolarization and channel formation, production of reactive oxygen species, release of cytochrome c, caspase activation, and cleavage of the nuclear enzyme poly(ADP-ribose) polymerase. UDCA may additionally be a strong activator of the mitogen-activated protein kinase survival pathway. Finally, it was recently reported that administration of TUDCA is neuroprotective not only in chemical-induced and transgenic animal models of Huntington's disease, but also for acute ischemic stroke. Intravenous administration of TUDCA after middle cerebral artery occlusion in the rat protected against cell death, reduced the infarct size and improved neurological function, while significantly decreasing mitochondrial membrane perturbation and downstream activation of caspases associated with apoptosis.

Rodrigues et al. (Journal of Cerebral Blood Flow & Metabolism (2002) 22: 463-71) suggest TUDCA in the treatment of ischemic stroke. The model used for the experiments is a rat model of transient focal cerebral ischemia. Matsushita et a. (Journal of Cerebral Blood Flow & Metabolism (2000) 20: 396-404) indicate that apoptosis may play a role in intracerebral hemorrhage induced in rats.

Neither surgical nor medical therapy has been effective at reducing morbidity or mortality after hemorrhagic stroke, and clinical trials in these patients have lagged far behind those for patients with ischemic stroke. There is an urgent need for therapeutic agents to mitigate the extensive cell death associated with hemorrhagic stroke and other injuries of the nervous system associated with hemorrhage.

### SUMMARY

The present invention provides uses of tauroursodeoxycholic acid as characterized in the claims. Preferably, the patient is a human patient,

One aspect of the present invention provides a use, wherein an effective amount of tauroursodeoxycholic acid, is to be administered . Preferably, administering comprises administering parenterally or orally.

Herein, a "patient" includes humans, sheep, horses, cattle, pigs, dogs, cats, and the like. Preferably, the subject is a human or other mammal

### BRIEF DESCRIPTION OF THE DRAWINGS

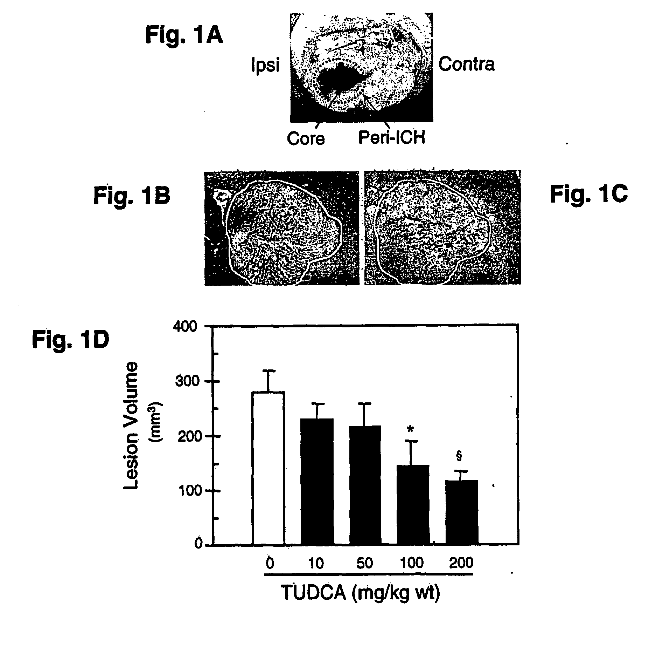

Figures 1A-1D - Collagenase-induced intrastriatal hemorrhage in rats results in a large hemorrhagic-lesion in the right striatum, at 2 days after collagenase infusion. (1A) Hemorrhagic lesion after coronal section through the rat brain subjected to experimental ICH. Outlined are the ipsilateral (Ipsi) ICH core and its periphery (Peri-ICH). Homologous tissue of contralateral (Contra) hemisphere was analyzed as a control. (1B) and (1C) The total lesion volume was markedly reduced in animals receiving increasing doses of TUDCA (10-200 mg/kg bw) administered 1 hour before collagenase. Representative Nissl-stained striatal sections are shown for each treatment group. The vehicle-injected striatal lesion (1B) is outlined for reference and superimposed on the striatum of TUDCA-treated animals (1C). (1D) Quantitation of lesion volumes are represented graphically as mean ± SEM (n=5 animals per group). **p* < 0.05 and §*p* < 0.01 from ipsilateral in vehicle-injected animals.

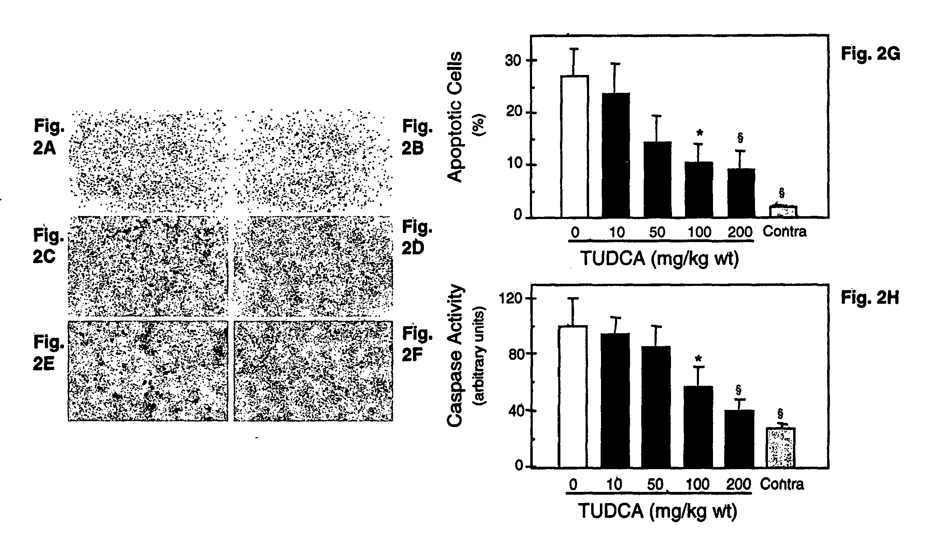

Figures 2A-2H - TUDCA reduces apoptosis in the hemisphere ipsilateral to ICH. (2A)-(2F) Representative striatal photomicrographs of TUNEL-stained sections. Rare TUNEL-positive cells were seen in the contralateral hemisphere in vehicle- (2A) and 100 mg/kg bw TUDCA-treated animals (2B). A marked increase in apoptotic cells was seen in the hemisphere ispilateral to the ICH in vehicle-injected animals (2C), and at higher magnification (2E). TUDCA-treated rats had significantly less TUNEL reactivity than sham-operated controls (2D), which could also be observed at higher magnification (2F). Sections are counterstained with 0.5% methyl green. Original magnification, 200x. (2G) Quantitation of apoptotic cells is indicated in the bar graph. Vehicle-injected rats exhibited increased proportions of apoptotic versus total cells compared with animals receiving increasing doses of TUDCA (10-200 mg/kg bw) administered 1 hour before collagenase. Data are presented as the mean ± SEM (n = 5 animals per group). (2H) DEVD-specific caspase activity is indicated in the bar graph. Activation of caspase-3 like proteases was significantly greater in the ipsilateral hemisphere of vehicle-injected controls compared with the contralateral hemisphere, but increasing dose of TUDCA progressively decreased caspase activation. Data are presented as the mean ± SEM (n = 5 animals per group). **p* < 0.05 and §*p* < 0.01 from ipsilateral in vehicle-injected animals.

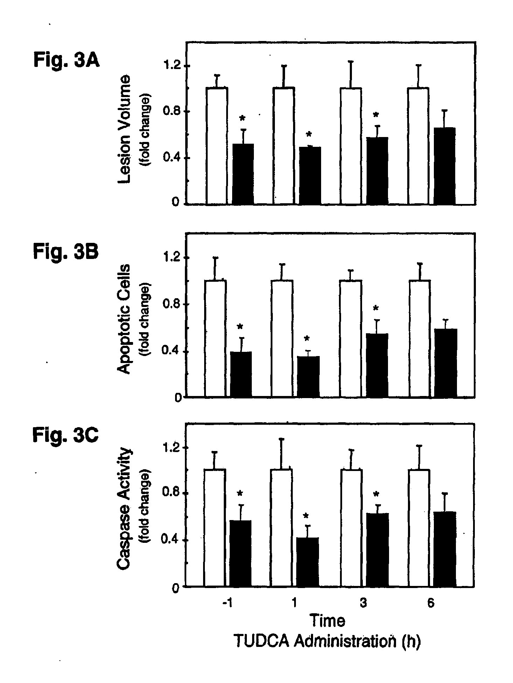

Figures 3A-3C - (3A) TUDCA (100 mg/kg bw) reduced lesion volumes, (3B) number of TUNEL-positive cells, and (3C) caspase activation in the ipsilateral hemisphere of ICH, when administered 1 hour before or at least up to 3 hours after the hemorrhagic incident. Data are presented as the mean ± SEM (n = 3-5 animals per broup). **p* < 0.05 and §*p* < 0.01 from ipsilateral in vehicle-injected animals.

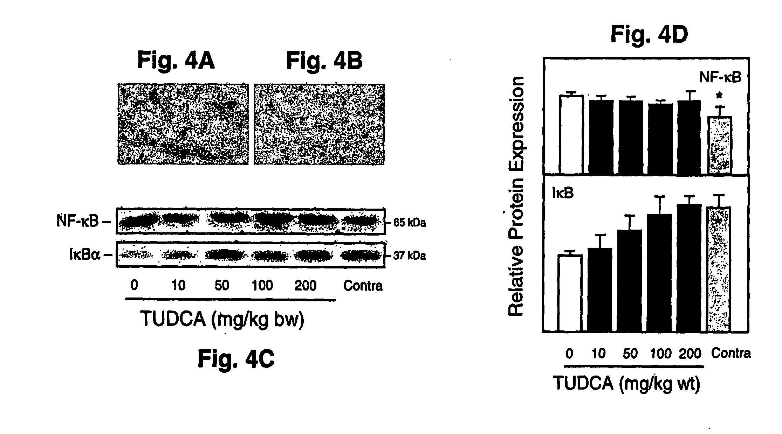

Figures 4A-4D - TUDCA treatment reduces NF-κB activation in the hemisphere ipsilateral to ICH. (4A) and (4B) Representative striatal sections showing activation of NF-κB/p65 in ipsilateral striatum 2 days after ICH. Immunoreactive NF-κB was clearly detectable in vehicle-injected controls (4A) but less evident in 100 mg/kg bw TUDCA-treated animals (4B). (4C) Representative immunoblot of p65 NF-κB subunit and IκBα in the ipsilateral hemisphere. Changes in NF-κB/IκBα protein-complex were visible in the ipsilateral hemisphere of sham-operated rats and less evident after increasing doses of TUDCA (10-200 mg/kg bw) administered 1 hour before collagenase. Total protein extracts were subjected to SDS-PAGE, and the blots probed with antibodies to NF-κB/p65 and IκBα. (4D) Quantitation of changes in NF-κB/IκBα protein complex are represented graphically as mean ± SEM (n = 3-5 animals per group). **p* < 0.05 from ipsilateral in vehicle-injected animals.

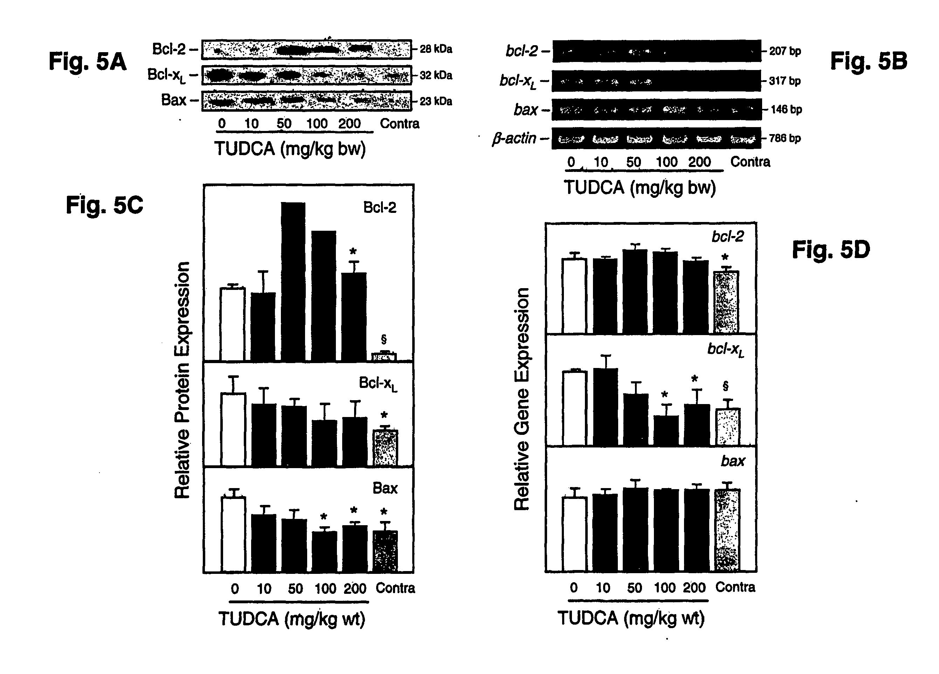

Figures 5A-5D - TUDCA treatment influences Bcl-2 family protein production and/or stability in the hemisphere ipsilateral to ICH. (5A) Bcl-2, Bcl-X_{L}, and Bax protein were elevated in the brain of vehicle-injected rats compared with the contralateral hemisphere. Increasing doses of TUDCA (10-200 mg/kg bw), administered 1 hour before collagenase, progressively diminished Bcl-xL and Bax, but not Bcl-2, production and/or stability. Representative immunoblot of Bcl-2, Bcl-x_{L}, and Bax and quantitation of respective protein levels. Total protein extracts were subjected to SDS-PAGE, and the blots probed with antibodies to Bcl-2 family members. (5B) mRNA levels of *bcl-2, bcl-x_{L},* and *bax* were elevated in the brain of vehicle-injected rats compared with the contralateral hemisphere. Increasing doses of TUDCA (10-200 mg/kg bw), administered 1 hour before collagenase, progressively diminished *bcl-x_{L},* but not *bcl-2,* transcriptional activation. Total RNA was isolated, and *bcl-2, bcl-x_{L},* and *bax* mRNA was amplified by RT-PCR. Amplified PCR products were analyzed by agarose gel electrophoresis and ethidium bromide staining. The product of constitutively expressed β-actin mRNA served as control. Data are presented as mean ± SEM (n = 3-5 animals per group) (5C) and (5D). **p* < 0.05 and §p < 0.01 from ipsilateral in vehicle-injected animals.

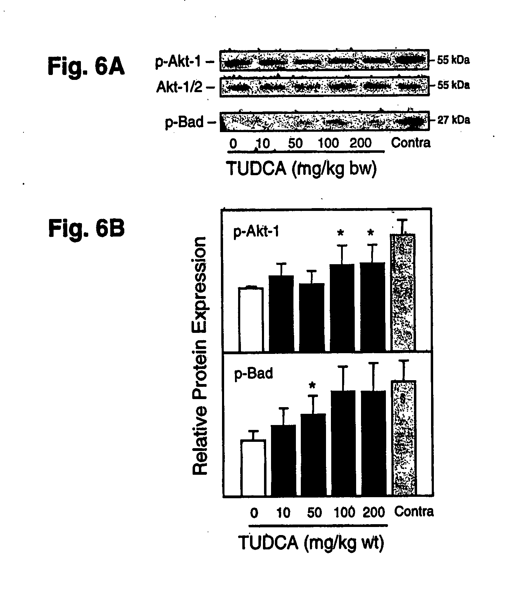

Figures 6A-6B - TUDCA activates Akt to protect neurons in the hemisphere ipsilateral to ICH. Phosphorylated Art-1 Ser-473 and phosphorylated Bad Ser-136 were diminished in the brain of vehicle-injected rats compared with the contralateral hemisphere. Increasing doses of TUDCA (10-200 mg/kg bw), administered 1 hour before collagenase, progressively increased Akt and Bad phosphorylation. Representative immunoblot of p-Akt-1, Akt-1/2, and p-Bad and quantitation of respective protein levels are shown in Fig. 6A. Total protein extracts were subjected to SDS-PAGE, and the blots probed with antibodies to phosphorylated Akt and Bad. Data are presented as mean ± SEM (n = 3-5 animals per group) (6B). **p* < 0.05 and §*p* < 0.01 from ipsilateral in vehicle-injected animals.

### DETAILED DESCRIPTION OF ILLUSTRATIVE EMBODIMENTS

The present invention provides the use of an effective amount of tauroursodeoxycholic acid for the preparation of a pharmaceutical composition for treating a patient having a hemorrhagic stroke and for reducing hematoma volume in a patient having suffered a hemorrhagic stroke as characterized in the claims.Also described herein is a method for treating a patient having a nervous system injury associated with hemorrhage. At present, there are no effective treatments to reduce the cell death associated with hemorrhage.

A nervous system injury is an injury of the nerves, which is characterized by the presence of hemorrhage. For example, the nervous system injury associated with hemorrhage could be a hemorrhagic stroke, a head trauma, a spinal cord injury, or a peripheral nerve injury.

Hemorrhagic stroke, is characterized by an actual bleed into the brain matter due to breakdown of blood vessel integrity. This is distinct from an ischemic stroke, which is characterized primarily by obstruction of blood flow into a prescribed area of the brain. Typically, ischemic strokes do not have associated hemorrhage but can be associated with edema in the area.

Although the compounds described herein are known for the treatment of ischemic stroke it was unexpected that they could be used for the treatment of hemorrhagic stroke or other nervous system injuries because of the inherent injury associated with hemorrhagic stroke. It was not known whether cell death was associated with apoptosis, necrosis and/or necroptosis. Immediate cell death (which typically occurs in hemorrhagic stroke) is typically a result of necrosis, whereas delayed cell death (which typically occurs in ischemic stroke) is typically a result of apoptosis.

The methods described herein involve the use of a hydrophilic bile acid, salts thereof, analogs thereof, or combinations thereof. As used herein, hydrophilic bile acids are those more hydrophilic than deoxycholic acid (DCA). This can be determined by evaluating the partition coefficient between water and octanol, with the more hydrophilic bile acids being more favorable toward water. Alternatively, the more hydrophilic bile acids have earlier retention times on a reverse-phase column using high performance liquid chromatography. A particularly suitable hydrophilic bile acid includes ursodeoxycholic acid. Examples of analogs of hydrophilic bile acids include suitable conjugated derivatives of bile acids. Two particularly suitable conjugated derivatives include glyco- and tauro-ursodeoxycholic acid. Other conjugated derivatives include ursodeoxycholic acid 3-sulfate, usrodeoxycholic acid 7-sulfate, and ursodeoxycholic acid 3,7-sulfate.

Although all hydrophilic bile acids may not be useful in all methods described herein, they can be evaluated readily by testing their ability to inhibit apoptosis in cell cultures systems using agents known to induce apoptosis.

Such compounds are used in amounts effective to treat a nervous system injury, which means both prophylactic and therapeutic treatments. Preferably, the treatment is a therapeutic treatment. For example, this applies to patients with hemorrhagic stroke, head trauma from car injuries, for example, peripheral nerve injuries associated with the job, and so on. Treatment will typically begin as soon as possible to reduce the impending cell death. The prophylactic use would relegate primarily to patients who may be at high risk for hemorrhagic stroke, such as a severely hypertensive patient.

An "effective amount" of tauroursodeoxycholic acid (TUDCA) for use in the present invention is such that the dosage level will be effective to prevent, reduce, inhibit, or suppress the development of damage as a result of hemorrhage.

TUDCA can be used in the methods of the present invention in the form of a composition that also includes a pharmaceutically acceptable carrier, if so desired. Typically, for preferred embodiments, TUDCA is formulated in pharmaceutical compositions and then, in accordance with the use of the invention, administered to a patient, typically a mammal, such as a human patient, in a variety of forms adapted to the chosen route of administration. The formulations include those suitable for oral, rectal, vaginal, topical, nasal, ophthalmic, or parental (including subcutaneous, intramuscular, intraperitoneal, intravenous, intrathecal, intraventricular, direct injection into brain tissue, etc.) administration.

The formulations may be conveniently presented in unit dosage form and may be prepared by any of the methods well known in the art of pharmacy. All methods include the step of bringing the active compound into association with a carrier, which can include one or more accessory ingredients. In general, the formulations are prepared by uniformly and intimately bringing the active compound into association with a liquid carrier, a finely divided solid carrier, or both, and then, if necessary, shaping the product into a desired formulation.

Formulations of the present invention suitable for oral administration may be presented as discrete units such as tablets, troches, capsules, lozenges, wafers, or cachets, each containing a predetermined amount of the apoptosis limiting compound as a powder, in granular form, incorporated within liposomes, or as a solution or suspension in an aqueous liquid or non-aqueous liquid such as a syrup, an elixir, an emulsion, or a draught.

The tablets, troches, pills, capsules, and the like may also contain one or more of the following: a binder such as gum tragacanth, acacia, corn starch or gelatin; an excipient such as dicalcium phosphate; a disintegrating agent such as com starch, potato starch, alginic acid and the like; a lubricant such as magnesium stearate; a sweetening agent such as sucrose, fructose, lactose or aspartame; and a natural or artificial flavoring agent. When the unit dosage form is a capsule, it may further contain a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac, or sugar and the like. A syrup or elixir may contain one or more of a sweetening agent, a preservative such as methyl- or propylparaben, an agent to retard crystallization of the sugar, an agent to increase the solubility of any other ingredient, such as a polyhydric alcohol, for example glycerol or sorbitol, a dye, and flavoring agent. The material used in preparing any unit dosage form is substantially nontoxic in the amounts employed. The compound may be incorporated into sustained release preparations and devices.

The compounds suitable for use in the invention can be incorporated directly into the food of a patient's diet, as an additive, supplement, or the like. Thus, a food product is also described herein. Any food is suitable for this purpose, although processed foods already in use as sources of nutritional supplementation or fortification, such as breads, cereals, milk, and the like, may be more convenient to use for this purpose.

Formulations suitable for parenteral administration conveniently comprise a sterile aqueous preparation of the desired compound, or dispersions of sterile powders comprising the desired compound, which are preferably isotonic with the blood of the recipient. Isotonic agents that can be included in the liquid preparation include sugars, buffers, and salts such as sodium chloride. Solutions of the desired compound can be prepared in water, optionally mixed with a nontoxic surfactant. Dispersions of the desired compound can be prepared in water, ethanol, a polyol (such as glycerol, propylene glycol, liquid polyethylene glycols, and the like), vegetable oils, glycerol esters, and mixtures thereof. The ultimate dosage form is sterile, fluid, and stable under the conditions of manufacture and storage. The necessary fluidity can be achieved, for example, by using liposomes, by employing the appropriate particle size in the case of dispersions, or by using surfactants. Sterilization of a liquid preparation can be achieved by any convenient method that preserves the bioactivity of the desired compound, preferably by filter sterilization. Preferred methods for preparing powders include vacuum drying and freeze drying of the sterile injectible solutions. Subsequent microbial contamination can be prevented using various antimicrobial agents, for example, antibacterial, antiviral and antifungal agents including parabens, chlorobutanol, phenol, sorbic acid, thimerosal, and the like. Absorption of the desired compounds over a prolonged period can be achieved by including agents for delaying, for example, aluminum monostearate and gelatin.

Nasal spray formulations can include purified aqueous solutions of the desired compound with preservative agents and isotonic agents. Such formulations are preferably adjusted to a pH and isotonic state compatible with the nasal mucous membranes. Ophthalmic formulations are prepared by a similar method to the nasal spray, except that the pH and isotonic factors are preferably adjusted to match that of the eye. Formulations for rectal or vaginal administration may be presented as a suppository with a suitable carrier such as cocoa butter, or hydrogenated fats or hydrogenated fatty carboxylic acids.

Topical formulations can include the desired compound dissolved or suspended in one or more media such as mineral oil, petroleum, polyhydroxy alcohols or other bases used for topical pharmaceutical formulations. Examples of such formulations include cosmetic lotion, crème, or sunscreen for use on the skin.

In addition to the aforementioned ingredients, the formulations used in the invention may further include one or more accessory ingredients including diluents, buffers, binders, disintegrants, surface active agents, thickeners, lubricants, preservatives (including antioxidants) and the like.

Useful dosages of the desired compounds described herein can be determined by comparing their *in vitro* activity and the *in vivo* activity in animals models. Methods for extrapolation of effective dosages in mice, and other animals, to humans are known in the art.

Generally, for adult humans, dosages for injection, infusion, or ingestion will generally vary from about 200 mg/day to about 7000 mg/day (i.e., a dosage of about 15 mg to 50 mg per kg of body weight per day). It may be administered, for example, about 1 to about 3 times per day, to yield levels of about 10 to about 15 µmol per liter or higher of serum. This could be a single bolus injection and then a continuous drip for 24-48 hours depending on the injury.

Advantages of the invention are illustrated by the following examples.

### EXAMPLES

In the following examples, the role of apoptosis in the collagenase-induced ICH model is further characterized and the nature and mechanisms of TUDCA-induced neuroprotection delineated. Specifically, TUDCA treatment led to a marked reduction in neuronal cell death and degeneration. Further, NF-κB activation was significantly decreased, Bcl-2 levels remained elevated, and rats showed improved neurological function. Additionally, TUDCA treatment activates Akt and induces Bad phosphorylation, thus promoting cell survival. Thus, it can be concluded that compounds such as TUDCA are unique, non-toxic endogenous, and potentially powerful, therapeutic agents for the treatment of hemorrhagic stroke and perhaps other apoptosis-associated injuries to the brain.

### MATERIALS AND METHODS

### Rat model of ICH

All animals received humane care in compliance with the institute's guidelines as outlined in "Guide for the Care and Use of Laboratory Animals" prepared by the National Academy of Sciences and published by the National Institutes of Health (NIH publication no. 86-23, revised 1985). Female Sprague-Dawley rats (250-300-grams (g); Harlan Sprague-Dawley, Inc., Indianapolis, IN) were anesthesized with an intramuscular injection of a mixture of ketamine and xylazine and set in supine in a Kopf headholder. The right carotid artery, external and internal branches, was exposed and the distal end of the external carotid freed and retracted to line with the internal carotid. A PE-10 polyethylene tube (Becton-Dickinson, Sparks, MD) was then introduced from the external into the internal carotid artery, and the former was ligated to hold the tube. TUDCA (Calbiochem-Novabiochem Corp., San Diego, CA) was dissolved in phosphate buffer, pH 7.4 at 400 milligrams per milliliter (mg/ml). Saline or TUDCA solution (1 milliliter per kilogram body weight (ml/kg bw)) were injected directly into the internal carotid artery, within 5 minutes. Different concentrations of TUDCA (10, 50, 100, and 200 mg/kg bw) were administered 1 hour prior to, or 1, 3, and 6 hours after collagenase injection. The polyethylene tube was finally removed, the end of the external carotid artery cauterized, and the wound closed. To induce stroke injury, the animals were placed in a stereotaxic apparatus and ICH induced by collagenase infusion *(*Rosenberg et al., Stroke 1990; 21: 801-807 *and* Chesney et al., Stroke 1995;26:312-316*).* In brief, a midline incision was made through the scalp to expose the skull. A 1-µl injection (0.5 microliters per minute (µl/min)) of saline containing 0.5 unit (U) bacterial collagenase (type VII; Sigma Chemical Co., St. Louis, MO) was made stereotaxically into the striatum with a 10-µl Hamilton syringe at the following coordinates: 0.4 millimeter (mm) anterior and 3.0 mm lateral to bregma and 5.0 mm ventral to the cortical surface. Once the infusion was complete, the Hamilton syringe was left in place for 3 minutes. Body temperature was maintained at 37°C with a CMA/150 temperature controller throughout the surgery. The rats recovered from surgery in a cage containing food and heated by an incandescent light bulb. Neurological testing was performed at 2 days after induction of ICH. The animals were then sacrificed, and the brains removed and frozen at 70°C for either serial cryostat-scctioning, or RNA and protein extraction. In a subset of animals, serum and brain samples were collected for bile acid analysis.

### Neurological testing

The animals were tested for rotational behavior in response to apomorphine 2 days after surgery. Apomorphine (1 mg/kg bw; Sigma Chemical Co.) was injected subcutaneously, and rotations were determined by a computer-controlled Columbus Instruments Videomex-V system (Columbus, OH). Counts were taken every 5 minutes after injection and continued for 60 minutes. Step initiation was assessed at 2 days after ICH by independently analyzing stepping behavior in the left and right forelimbs. In this test, the animal is held in a manner that restrains one forelimb and both hind limbs. The free limb is placed on a flat surface so that the body weight of the animal is centered over the limb. The rat initiates a stepping movement, and the investigator adjusts the position of the rat so that the body weight of the animal is again centered over the limb. The number of steps initiated by each limb was counted during a 1-minute period.

### Hematoma volume

After behavioral testing, rats were sacrificed and the brains removed for serial cryostat sectioning. Volumes of hemorrhagic lesion, including core and peri-ICH regions, were quantitated using unbiased stereologic volumetric analysis of 20-µm cryostat sections after staining according to the Nissl protocol. Volumetric analysis was performed according to Cavalieri's method *(*Howard et al., Unbiased stereology: three-dimensional measurement in microscopy. New York: BIOS Scientific Publishers Ltd 1998*).* The areas of lesion were determined in 11 Nissl-stained sections 20 µm in thickness, 900 µm apart, from each animal. Volumes (mm³) were calculated according to the formula V = T x a/p x ΣPi, where V = volume of interest, T = distance between sections (mm), a/p = area associated with each point (mm²), and Pi = points hitting object in each section.

### TUNEL staining

At 2 days after collagenase infusion, rats were sacrificed for terminal deoxynucleotidyltransferase-mediated dUTP nick end labeling (TUNEL) of apoptotic cells. Brain sections 10 µm in thickness, were fixed with 1% formaldehyde, post-fixed with acetate-ethanol-solution at -20°C, and washed with phosphate buffer, pH 7.4. An Apoptag *in situ* apoptosis detection kit (Intergen Co., Purchase, NY) was used for TUNEL staining according to the manufacturer's recommendations. In brief, the same serial brain section for each animal was incubated with equilibration buffer. Terminal deoxynucleotidyltransferase and digoxigenin-dNTP were added to the sections and incubated at 37°C for 1 hour (h). Slides were then treated with anti-digoxigenin-peroxidase solution for 30 minutes (min), colorized with DAB substrate, and counterstained with 0.5% methyl green. The number of TUNEL-positive cells was counted on a computer screen grid from at least three random fields (400x) within the region adjacent to the hemorrhagic core for each animal.

### DEVD-specific caspase activity.

Caspase activation was determined in dissected brain tissue from the ispilateral and corresponding contralateral areas. The tissues were homogenized in isolation buffer containing 10 mM Tris-HCl buffer, pH 7.6,5 mM MgCl₂, 1.5 mM KAc, 2 mM DTT, and protease inhibitor cocktail tablets (COMPLETE; Roche Diagnostics, GmbH, Mannheim, Germany). General caspase activity was evaluated by enzymatic cleavage of chromophore *p*-nitroanilide (pNA) from the substrate *N*-acetyl-Asp-Glu-Val-Asp-pNA (DEVD-pNA; Sigma Chemical Co.). The proteolytic reaction was carried out in isolation buffer containing 50 µg cytosolic protein and 50 µM DEVD-pNA. The reaction mixtures were incubated at 37°C for 1 hour, and the formation of pNA was measured at 405 nm using a 96-well plate reader. Protein concentrations were determined using the Bio-Rad protein assay kit (Bio-Rad Laboratories, Hercules, CA) according to the manufacturer's specifications.

### RNA Isolation and RT-PCR

Changes in Bcl-2 family-expression were determined by RT-PCR. Total RNA was extracted from brain tissue using the TRIZOL reagent from Life Technologies, Inc. (Grand Island, NY). For RT-PCR, 5 µg of total RNA was reverse-transcribed using oligo(dT) (IDT, Inc., Coralville, IA) and SUPERSCRIPT II reverse transcriptase (Life Technologies Inc.). Specific oligonucleotide primer pairs were incubated with cDNA template for PCR amplification using the Expand High Fidelity PCR System from Roche Diagnostics, GmbH. The following sequences were used as primers: Bcl-2 sense primer, 5'-CTGGTGGCAACATCGCTCTG-3' (SEQ ID NO: 1); and Bcl-2 antisense primer, 5'-GGTCTGCTGACCTCACTTGTG-3' (SEQ ID NO:2); Bax sense primer, 5'-TGGTTGCCCTTTTCTACTTTG-3' (SEQ ID NO:3); and Bax antisense primer, 5'-GAAGTAGGAAAGGAGGCCATC-3' (SEQ ID NO:4); Bcl-x sense primer, 5'-AGGTCGGCGATGAGTTTGAA-3' (SEQ ID .NO:5) ; and Bcl-x antisense primer, 5'-CGGCTCTCGGCTGCTGCATT-3' (SEQ ID NO:6); β-actin sense primer, 5'-TGCCCATCTATGAGGGTTACG-3' (SEQ ID NO: 7), and β-actin antisense primer, 5'-TAGAAGCATTTGCGGTGCACG-3' (SEQ ID NO:8). The product of constitutively expressed β-actin mRNA served as control. Amplified PCR products were analyzed by agarose gel electrophoresis and ethidium bromide staining.

### Immunoblotting and immunohistochemistry

Bcl-2 family, NF-κB, IκB, p-Akt, and p-Bad protein levels were determined from total protein homogenates separated by 12% sodium dodecyl sulfate polyacrylamide gel electrophoresis (SDS-PAGE). Blots were probed with either primary mouse monoclonal antibodies reactive with Bcl-2 and NF-κB, or with primary rabbit polyclonal antibodies reactive to Bcl-x_{S/L}, Bax, IκB, p-Akt, and p-Bad at a dilution of 1:500 (Santa Cruz Biotechnology, Santa Cruz, CA), and subsequently incubated with secondary anti-mouse or anti-rabbit antibodies conjugated with horseradish peroxidase. Finally, membranes were processed for specific protein detection using the SUPERSIGNAL substrate (Pierce, Rockford, IL). Immunohistochemistry of NF-κB activation was performed in coronal cryosections 10 µm in thickness fixed with 1% formaldehyde, post-fixed with acetate-ethanol solution at -20°C, and washed with phosphate buffer, pH 7.4, using the IMMUNOCRUZ staining system (Santa Cruz Biotechnology), according to the manufacturer's instructions. In brief, after blocking with normal goat serum, the cryostat sections were probed with an activation specific monoclonal antibody for NF-κB p65 subunit (Santa Cruz Biotechnology), the epitope of which is available for binding only after IκB dissociation. Secondary anti-mouse antibody conjugated with biotin was applied and the slides were then treated with HRP-streptavidin complex, colorized with HRP substrate mixture, and counterstained with 0:5% methyl green. Multiple sections from each rat were examined in the region adjacent to the hematoma.

### Bile acid analysis of serum and brain

Normal controls and rats with intrastriatal hematoma were sacrificed 1,3, and 6 hours after TUDCA or vehicle injection. Blood was collected, clotted, spun, and the serum removed and frozen at -20°C. In addition, brains were removed following 5% chloral hydrate anesthesia and transcardial phosphate buffer perfusion, flash frozen and stored at -70°C. Bile acids were assessed by gas chromatography after organic solvent extraction, purification on Lipidex 1000, liquid-solid extraction, hydrolysis, isolation by lipophilic anion exchange chromatography and conversion to methyl ester-trimethylsilyl ether derivatives *(*Setchell et al., Gastroenterology 1997;112:226-235*).* Bile acid concentrations were expressed as nmol/g of tissue or as µmol/l of serum.

### Densitometry and statistical analysis

The relative intensities of the protein and nucleic acid bands were analyzed using the ImageMaster 1D Elite densitometric analysis program (Amersham Pharmacia Biotech, Uppsala, Sweden). Statistical analysis was performed using GraphPad InStat version 3.00 for Windows 95 (GraphPad Software, San Diego, CA) for the ANOVA and Bonferroni's multiple comparison tests. Values of p < 0.05 were considered as significant.

### RESULTS

TUDCA decreases hematoma volumes and apoptosis after hemorrhagic stroke. ICH that recapitulates damage caused by human stroke can be induced in adult rats by injecting bacterial collagenase into the brain parenchyma, where it causes extensive disruption of blood vessels. See-Figures 1A-1D. Computerized volumetric analysis of Nissl staining within serial sections revealed a large hemorrhagic lesion in the right striatum, at 2 days after collagenase infusion (Fig. 1A). The volume of the lesion correlated with the degree of striatum atrophy, whereas the decreased volume in the ipsilateral striatum was compensated by an increase in ipsilateral ventricular size (data not shown). In addition, the lesion size was markedly reduced in animals receiving increasing doses of TUDCA administered 1 hour before collagenase infusion. The hemorrhagic lesions of the brains were found to be approximately 50% larger in the vehicle-injected group, compared with 100 mg/kg bw TUDCA-treated rats (278.4 ± 39.2 mm³ vs. 143.9 ± 46.5 mm³ in vehicle vs. treatment group, respectively; *p* < 0.05). At 200 mg/kg bw TUDCA, an approximate 60% reduction in lesion volume was also observed (*p* < 0.01), whereas 10 and 50 mg/kg bw dose regimens were significantly less protective. Finally, brains of animals receiving TUDCA exhibited a less pronounced presence of inflammatory cells outside of the hemorrhagic core (data not shown).

TUNEL staining was used to identify cells with nuclear DNA fragmentation. Few apoptotic cells were observed in the contralateral hemisphere in both experimental groups (Figs. 2A-2G). However, TUNEL-positive cells were markedly increased in the ipsilateral hemisphere of sham-operated rats (*p* < 0.01). Electrophoresis of DNA extracted from the lesioned hemisphere of hemorrhagic brains also showed evidence of characteristic DNA laddering into approximately 200-bp fragments (data not shown). TUDCA treatment reduced the number of apoptotic cells from almost 30% to approximately 10% after 100 mg/kg bw-(*p* < 0.05). In addition, when TUDCA was administered at greater dosages, it reduced apoptosis by > 65% (*p* < 0.01). Caspase activity in both the ipsilateral and contralateral hemispheres after collagenase infusion was also examined. Protein extracts were prepared from relevant brain tissues and incubated with DEVD-pNA, a preferred substrate for caspase-3-like enzymes. There was no significant increase in caspase activity in the contralateral hemispheres compared to normal brain tissue (data not shown). In contrast, caspase activity was almost 4-fold increased in the ipsilateral portion of the brain in vehicle-injected rats (*p* < 0.01) (Fig. 2H). TUDCA reduced caspase activation in the lesioned hemisphere by 45-60% after 100 and 200 mg/kg bw TUDCA administration (*p* < 0.05 and *p* < 0.01, respectively). In fact, there were no statistical differences between the highest dose TUDCA-treated ipsilateral and contralateral regions. These findings suggest that, in the collagenase model of hemorrhagic stroke, neurons outside of the core region are dying by apoptosis and can be efficiently rescued by TUDCA.

Next, the therapeutic window of TUDCA administration was determined. Using the same model, vehicle or TUDCA at 100 mg/kg bw was injected 1 hour before, or 1, 3, and 6 hours after collagenase infusion. The volume of brain lesioned 2 days after the hemorrhage was markedly reduced in animals receiving TUDCA administered 1 hour before or up to 3 hours after the hemorrhagic incident (*p* < 0.05) (Fig. 3A). By 6 hours, this 40-50% protective effect was partially lost but still evident. Peri-ICH volumes, which represented up to 72% of the total hemorrhagic lesion, were significantly reduced by TUDCA at any time point (*p* < 0.05) (see Table 1 below). However, TUDCA provided no apparent protection in the core lesion volumes when given 6 hours after the hemorrhage, suggesting that the effect by TUDCA was stronger outside of the small hemorrhagic core and more evident when administered earlier. TUNEL staining and caspase activity remained markedly lower in TUDCA-treated than in vehicle-injected animals throughout the time-course experiment (Figs. 3B and 3C). Thus, in this model of stroke, a window at least up to 3 hours after hemorrhage appears open for intervention by using TUDCA, as animals receiving TUDCA at 1 hour or 3 hours, revealed a similar protective effect as pretreatment.

**Table 1- Reduction of ICH core and its periphery (Peri-ICH) by TUDCA**

| | Protection (%) | |
|---|---|---|
| Time TUDCA administration (h) | ICH core | Peri-ICH |
| 1 | 47.7 ±3.2* | 48.6 ±3.5* |
| 3 | 30.0 ±18.9* | 42.8 ± 14.9* |
| 6 | 20.9 ± 12.9 | 38.7 ± 12.9* |

| | | |
|---|---|---|
| Data are presented as mean ± SEM (n = 3-5 animals per group). **p* < 0.05 from vehicle-injected animals, at the respective time point. | | |

Bile acid analysis showed that arterial administration of TUDCA resulted in increased concentrations in serum and brain. In fact, a single 100 mg/kg bw dose of TUDCA resulted in a significant elevation of the bile acid in serum from 0.3 to 10.6 µmol/l (*p* < 0.01) at 3 hours following injection. This change was associated with a 10-fold increase in brain concentrations of this bile acid compared with vehicle-injected controls (*p* < 0.01). In addition, among the rats receiving TUDCA, there was an almost 2-fold elevation in brain total bile acid concentrations between the animals with stroke and normal controls (*p* < 0.05), suggesting increased transport across the blood-brain barrier.

### TUDCA ameliorates neurological deficits

Functional neurological deficits 2 days after hemorrhage were improved in rats treated with TUDCA, indicating that lesion sparing may be accompanied by improved neurofunction. A strong net ipsilateral rotation in response to apomorphine Administration was noted in the vehicle-injected group at 2 days after surgery. TUDCA administration, in contrast, attenuated the-general responses of motor activity. In fact, the rotational response decreased from approximately 20 per 5 minutes in vehicle-injected controls to less than 10 in rats treated with TUDCA (*p* < 0.05). The protective effect was more evident during earlier administrations but still significant when TUDCA was given 6 hours after ICH. The ability of an animal to place the limb and to initiate stepping movements was also assessed. The test of limb placement from a variation of De Ryck (De Ryck. Eur. Neurol. 1990; 30(suppl. 2):21-27) showed a 25-40% improved motor activity through the time-course experiment of TUDCA administration. Indeed, during a 1-minute period, the left forelimb initiated significantly more steps in animals with unilateral striatal hemorrhage treated with TUDCA than in vehicle-injected rats (*p* < 0.05). In addition, the difference between the right and left forelimbs was smaller in rats that received TUDCA, either at 1 hour before or 1 hour after collagenase infusion than in rats treated at later time points.

### NF-κB activation is decreased in TUDCA-treated rats

To explore signaling pathways in hemorrhagic stroke and the protective role of TUDCA, brain tissue adjacent to the core lesion, where protein synthesis is still possible, as well as homologous tissue of contralateral hemisphere as a control were used. See Figures 4A-4D. Changes in NF-κB/IκBα protein complex, a major sensor and effector of oxidative stress in cells, were very marked in the ipsilateral hemisphere of sham-operated rats and significantly less evident after TUDCA treatment. Western blot analysis for IκBα showed an almost 40% decrease in vehicle-injected controls compared with the contralateral hemisphere (*p* < 0.05), but could be largely restored by treatment with increasing doses of TUDCA. Moreover, in sham-operated rats, NF-κB p65 subunit expression was significantly increased (*p* < 0.05), whereas TUDCA-injected animals revealed no further difference (Figs. 4C and 4D). To confirm NF-κB activation after ICH, immunohistochemistry, in which an activation-dependent anti-NF-κB antibody was used. Immunoreactive NF-κB in the ipsilateral hemisphere was clearly detectable in sham control animals, whereas TUDCA treatment markedly decreased NF-κB activation in the peri-ICH region (Figs. 4A and 4B). Further, the distribution area of NF-κB activation coincided with that of DNA fragmentation measured by TUNEL staining (data not shown).

Previous reports have demonstrated that Bcl-2 functions to inhibit cytochrome c release from mitochondria to cytosol and subsequent cell death *(*Kluck et al., Science 1997; 275:1132-1136 and Yang et al., Science 1997; 275:1129-1132*).* Therefore, whether intrastriatal hemorrhage induces Bcl-2 protein and other family elements, Bcl-x and Bax, in relation to NF-κB activation was examined. See Figures 5A-5D. Western blots revealed a very pronounced increase of approximately 6-fold in Bcl-2 protein levels in the ipsilateral hemisphere following collagenase infusion (*p* < 0.01), and a less evident elevation in Bcl-x_{L} (*p* < 0.05) (Figs. 5A and 5C). Pro-apoptotic Bax was also significantly increased (*p* < 0.05). Bcl-2 protein levels in the presence of TUDCA did not correlate with NF-κB activation, as Bcl-2 remained elevated, throughout the dose-response study, while activation of the transcription factor was markedly reduced. Bcl-x_{L} protein levels were slightly diminished by TUDCA, whereas Bax protein returned to contralateral hemisphere values during treatment. RT-PCR analysis revealed a significant increase in *bcl-2* (*p* < 0.05) and *bcl*-x_{L} (*p* < 0.01) mRNA levels following intrastriatal hemorrhage, whereas bax mRNA was unchanged (Figs. 5B and 5D). TUDCA further increased transcriptional activation of Bcl-2, but decreased *Bcl*-x_{L} mRNA. These findings suggest that, in the collagenase model of hemorrhagic stroke, up-regulation of bcl-2 and bcl-x_{L} expression is associated with NF-κB activation, which in turn is significantly decreased by TUDCA. Further, administration of TUDCA before ICH is associated with increased production and/or stability of Bcl-2, probably through an alternative pathway independent of NF-κB activation.

### TUDCA activates Akt to protect neurons after hemorrhagic stroke

Akt, the cellular homolog of the viral oncoprotein v-Akt suppresses apoptotic cell death in a number of cell types. Akt has also been implicated in the phosphorylation of Bad, potentially linking survival pathways with the Bcl-2 family. When exploring TUDCA signaling pathways, it was found that the expression of the phosphorylated form of Akt-1 was markedly decreased in the hemisphere ipsilateral to ICH (*p* < 0.01), but could be largely restored by treatment with TUDCA. See Figures 6A and 6B. Similarly, levels of phosphorylated Bad were almost 2-fold elevated in the brain of rats receiving TUDCA, compared with untreated sham controls (*p* < 0.05). Whether intracellular signaling through the phosphatidylinositide 3'-OH kinase (PI3K) pathway is critical for TUDCA, effects in neurons was evaluated by using a selective inhibitor of PI3K to block the phosphorylation of Akt *in vitro.* Treatment of primary cortical neurons with wortmannin partially inhibited TUDCA-induced Akt phosphorylation, while significantly decreasing cell viability (data not shown).

### DISCUSSION

UDCA and TUDCA are nontoxic, endogenously produced, bile acids that act both in vitro and in vivo as potent anti-apoptotic agents. In addition to their effects at preventing mitochondrial dysfunction by inhibiting membrane depolarization and channel formation *(*Rodrigues et al., J Clin Invest 1998;101:2790-2799 and Rodrigues et al., Cell Death Differ 1999;6:842-854*),* these molecules may also play key regulatory roles in signal transduction pathways by modulating cytosolic calcium levels and in gene expression by regulating transcription *(*Guicciari et al., Hepatology 2002;35:971-973*).* Administration of TUDCA to a genetic mouse model of Huntington's disease significantly reduced striatal degeneration and improved locomotor and sensorimotor deficits *(*Keene et al., Proc Natl Acad Sci USA 2002;99:10671-10676*).* Further, when given after ischemia in the middle-cerebral artery occlusion model of stroke, TUDCA significantly reduced apoptosis, infarct volumes, and neurobehavioral impairment *(*Rodrigues et al., J Cereb Blood Flow Metab 2002;22:463-471*).* The present study extends our previous reports and shows a neuroprotective effect of TUDCA in a collagenase-induced ICH model of stroke. Administration of TUDCA almost completely abolished the appearance of TUNEL-positive cells, the activation of caspase-3-like proteases, and the histological damage of the peri-ICH region. These findings are consistent with the inhibition of neuronal apoptosis by TUDCA. Further, most neurons can be rescued by TUDCA up to 3 hours, and some up to 6 hours, from the onset of the hemorrhage, consistent with a delayed activation of neuronal cell death. Thus, at least in this model, many cells remain viable for several hours and can be recovered by an anti-apoptotic agent, provided therapy is initiated within this time window. Finally, TUDCA was also markedly effective at reducing apoptosis after brain injury in a blunt head trauma rat model. In fact, the number of TUNEL-positive cells per high power field was approximately 27 in vehicle-injected animals with traumatic brain injury, but only 12 in rats receiving TUDCA. The more than 50% reduction of apoptosis by TUDCA was achieved after a 24-h pre-injury treatment, followed by two post-injury treatments at the time of head trauma and 24 h later.

ICH remains a devastating cerebrovascular event with an estimated incidence of 15 to 35 per 100,000 *(*Broderick et al., N Engl J Med 1992; 326: 733-736 and Hankey et al., Stroke 1997;28:2126-2132*)* and a mortality rate approaching 50% *(*Juvela, Arch Neurol 1995;52:1193-1200*).* Neither surgical nor medical therapy has been effective at reducing morbidity or mortality after ICH, and clinical trials in these patients have lagged far behind those for patients with ischemic stroke. Injury secondary to the mass-effect of the hematoma is thought to arise from tissue reaction to invasion of blood products, resulting in ischemia, edema, intense inflammation *(*Mendelow, Stroke 1993;24:1115-7*),* and ultimately cell death. The mechanisms of cell death from acute stroke are complex and appear to involve an interplay of events. However, it has been recognized that after cerebral ischemia followed by reperfusion, cells exhibit several features of apoptosis including chromatin condensation, TUNEL labeling, and activation of caspases *(*Choi, Curr Opin Neurobiol 1996;6:667-672). Characteristic signs of apoptosis have also been described in the brain after hemorrhagic stroke *(*Gong et al., Neurosurgery 2001;48:875-882 and Matz et al., J Cereb Blood Flow Metab 2001; 21:921-928*).* Consistent with these observations, dramatically increased numbers of TUNEL-positive cells, and significant caspase activation in the region immediately surrounding the hematoma was detected. In fact, the evidence suggests that this narrow zone of selective neuronal injury, where various apoptosis-related proteins are expressed, could be important for stroke outcome. This is supported by recent studies where animals treated with caspase inhibitors and genetically engineered caspase-deficient mice showed reduced ischemic brain injury *(*Loddick et al., NeuroReport 1996; 7:1465-1468 and Hara et al., Proc Natl Acad Sci USA 1997;94:2007-2012 and Cheng et al., J Clin Invest 1998;101:1992-1999 and Endres et al., J Cereb Blood Flow Metab 1998;18:238-247 and Schielke et al., J Cereb Blood Flow Metab 1998;18:180-185 and Rabuffetti et al., J Neurosci 2000;20:4398-4404*).*

TUDCA, administered up to 3 hours after ICH, significantly reduced striatal lesion volumes, with associated improvement of neurological function, by preventing apoptotic cell death. TUDCA directly inhibits reactive oxygen species production, collapse of the membrane potential, and disruption of the outer mitochondrial membrane *(*Rodrigues et al., Mol Med 1998;4:165-178 and Rodrigues et al., Biochem Biophys Res Commun 2001;281:468-474*)*. Additionally, TUDCA mitigates mitochondrial insufficiency and toxicity by inhibiting Bax translocation from cytosol to mitochondria *(*Rodrigues et al., J Neurochem 2000; 75:2368-2379 and Rodrigues et al., Mol Med 1998;4:165-178 and Rodrig ues et al., Cell Death Differ 1999;6:842-854*).* Membrane stability inhibits cytochrome c release, thereby mediating downstream events such as caspase activation and substrate cleavage *(*Rodrigues et al., Mol Med 1998;4:165-178 and Rodrigues et al., Cell Death Differ 1999;6:842-854 and Benz et al., J Hepatol 1998;28:99-106*).*

It is now well established that NF-κB activation has a prominent role in the stroke-injured brain *(*Sharp et al., J Cereb Blood Flow Metab 2000;20:1011-1032*).* ICH is specifically accompanied by an early activation of NF-κB *(*Hickenbottom et al., Stroke 1999,30.-2472-2477*),* which may then increase transcription of a number of different genes including some involved in inflammation and other implicated in apoptosis. The precise role of NF-κB in stroke remains, however, unclear because some studies show that NF-κB might mediate injury, whereas others suggest that it could protect the brain. Although absolute amounts of p50 and p65 may not be predictive, NF-κB DNA binding activity is increased after experimental ICH and activation is frequently colocalized to cells containing fragmented DNA *(*Hickenbottom et al., Stroke 1999;30:2472-2477*),* suggesting a harmful role for NF-κB. Paradoxitally, reducing the expression of a key mediator of NF-κB activation, the proinflammatory cytokine tumor necrosis factor-α, trough the use of antisense oligodeoxynucleotides can be neuroprotective after ICH *(*Mayne, et al., Stroke 2001;32:240-248*).* Consistent with previous reports, significant activation of NF-κB in the region immediately surrounding the hematoma at 2 days after ICH was detected. Downstream target genes of NF-κB encoding for the anti-apoptotic protein Bcl-2, and to a lesser extent Bcl-x_{L}, were highly expressed in the same brain region, suggesting the activation of a survival pathway to protect the brain against the initial inflammatory response that contributes to cell injury. Interestingly, Bcl-2 levels continues to be elevated after TUDCA administration despite a significant decrease in NF-κB activation. Thus, in the setting of ICH, the initial inflammatory response and/or oxidative stress result in important NF-κB activation, which appears to protect the brain, as Bcl-2 and Bcl-x_{L}expression are increased, but may simultaneously exacerbate injury through the activation of other genes that mediate cell death. Indeed, apoptosis is still a prominent form of-cell death in the peri-ICH region. By preventing the initial injury, TUDCA, might indirectly decrease NF-κB activation. Simultaneously, Bcl-2 levels remain elevated probably through the activation of alternative survival pathways by TUDCA. This study confirms evidence that specific signaling cascades that have been characterized in hematopoietic cells and other cell types *(*Ihe, Nature 1995;37:591-594 and Siren et al., Proc Natl. Acad Sci USA 2001;98:4044-4049*)* are functional in the rat brain and can be modulated by TUDCA. More importantly, these pathways appear to be crucial for the neuroprotective effect of TUDCA. Akt phosphorylation by TUDCA is potentially linked with Bad phosporylation, which is known to promote survival by disrupting its ability to bind to and inactivate antiapoptotic Bcl-2 family members *(*Datta et al., Genes Dev 1999;133'2905-2927*).* Although it is not entirely clear from this study how TUDCA reduced NF-κB activation, and activated protein kinase pathways to alter the ability of apoptotic molecules to promote survival, the ability to revert neuronal injury is an important consideration in the treatment of hemorrhagic stroke.

## Claims

1. Use of an effective amount of tauroursodeoxycholic acid for the preparation of a pharmaceutical composition for treating a patient having a hemorrhagic stroke.

2. The use of claim 1, wherein the patient is a human patient.

3. The use according claim 1 or 2, wherein the pharmaceutical composition further comprises a pharmaceutically acceptable carrier.

4. The use according to anyone of claims 1 to 3, wherein the pharmaceutical composition is to be administered parenterally.

5. The use according to anyone of claims 1 to 3, wherein the pharmaceutical composition is to be administered orally.

6. The use according to anyone of claims 1 to 5, wherein the pharmaceutical composition is to be administered to the patient up to three hours after the injury.

7. The use according to anyone of claims 1 to 5, wherein the pharmaceutical composition is to be administered to the patient up to six hours after the injury.

8. Use of an effective amount of tauroursodeoxycholic acid for the preparation of a pharmaceutical composition for reducing hematoma volume in a patient having suffered a hemorrhagic stroke.

## Patentansprüche

1. Verwendung einer wirksamen Menge Tauroursodesoxycholsäure zur Herstellung einer pharmazeutischen Zusammensetzung zur Behandlung eines Patienten mit hämorrhagischem Schlaganfall.

2. Verwendung nach Anspruch 1, wobei der Patient ein menschlicher Patient ist.

3. Verwendung nach Anspruch 1 oder 2, wobei die pharmazeutische Zusammensetzung weiter einen pharmazeutisch verträglichen Träger umfasst.

4. Verwendung nach einem der Ansprüche 1 bis 3, wobei die pharmazeutische Zusammensetzung parenteral zu verabreichen ist.

5. Verwendung nach einem der Ansprüche 1 bis 3, wobei die pharmazeutische Zusammensetzung oral zu verabreichen ist.

6. Verwendung nach einem der Ansprüche 1 bis 5, wobei die pharmazeutische Zusammensetzung dem Patienten bis zu drei Stunden nach der Schädigung zu verabreichen ist.

7. Verwendung nach einem der Ansprüche 1 bis 5, wobei die pharmazeutische Zusammensetzung dem Patienten bis zu sechs Stunden nach der Schädigung zu verabreichen ist.

8. Verwendung einer wirksamen Menge Tauroursodesoxycholsäure für die Herstellung einer pharmazeutischen Zusammensetzung zur Reduktion des Hämatomvolumens in einem Patienten, der einen hämorrhagischen Schlaganfall erlitten hat.

## Revendications

1. Utilisation d'une quantité efficace d'acide tauroursodéoxycholique pour la préparation d'une composition pharmaceutique pour traiter un patient ayant une attaque hémorragique.

2. Utilisation selon la revendication 1, dans laquelle le patient est un patient humain.

3. Utilisation selon la revendication 1 ou 2, dans laquelle la composition pharmaceutique comprend en outre un support pharmaceutiquement acceptable.

4. Utilisation selon l'une quelconque des revendications 1 à 3, dans laquelle la composition pharmaceutique est à administrer par voie parentérale.

5. Utilisation selon l'une quelconque des revendications 1 à 3, dans laquelle la composition pharmaceutique est à administrer oralement.

6. Utilisation selon l'une quelconque des revendications 1 à 5, dans laquelle la composition pharmaceutique est à administrer au patient jusqu'à trois heures après la lésion.

7. Utilisation selon l'une quelconque des revendications 1 à 5, dans laquelle la composition pharmaceutique est à administrer au patient jusqu'à six heures après la lésion.

8. Utilisation d'une quantité efficace d'acide tauroursodéoxycholique pour la préparation d'une composition pharmaceutique pour réduire le volume de l'hématome chez un patient ayant souffert d'une attaque hémorragique.
